# EUROPÄISCHE PATENTSCHRIFT

(11) **EP 1 288 290 B1**
(45) Veröffentlichungstag und Bekanntmachung des Hinweises auf die Patenterteilung: **02.01.2008**
(21) Anmeldenummer: 02018004.8
(22) Anmeldetag: 12.08.2002
(51) Int. Cl.: C12N 1/14

(54) **Verfahren zum selektiven Nachweis von Hefen und Pilzen**
Method for the selective detection of yeast and fungi
Procédé de détection selective des levures et champignons

(30) Priorität: 14.08.2001 DE 10139965
(43) Veröffentlichungstag der Anmeldung: 05.03.2003
(73) Patentinhaber: Biotest AG, D-63303 Dreieich (DE)
(72) Erfinder: Horn, Jürgen, 63329 Egelsbach (DE)
(74) Vertreter: Strych, Werner Maximilian Josef

(56) Entgegenhaltungen:
- WO-A-93/03772
- DE-A- 3 539 393
- CHU T.W. ET AL.: "Structure-activity relationships of the fluoroquinolones" ANTIMICROBIAL AGENTS AND CHEMISTRY, Bd. 33, Nr. 2, 1989, XP000900357

## Beschreibung

Gegenstand der Erfindung ist ein Verfahren zum Selektiven Nachweis von Hefen und Pilzen mit den Merkmalen des Anspruchs 1.

Medien zum selektiven Nachweis von Hefen und Pilzen enthalten Antibiotika wie Penicillin und Streptomycin oder Chloramphenicol und Chlortetrazyklin (Air Quality Monographs Vol. 2, Elsevier Press 1994), um das gleichzeitige Wachsen von Bakterien zu verhindern. Der Zusatz dieser Antibiotika sowie einer Reihe weiterer Antibiotika zu Sabouraud-Medium wird bei McFaddin. Media for Isolation-Cultivation-Identification-Maintenance of Medical Bacteria, Williams & Wilkins, 1985 beschrieben.

Die deutsche Patentanmeldung Nr. 35 39 393 beschreibt die Verwendung von Ciprofloxacin zur Dekontamination bei Mycoplasma-infizierten Zellkulturen. Das Ciprofloxacin kann dabei in einer Menge von 1 bis 1000 mg/L vorhanden sein. Die Zellkulturmedien können gegebenenfalls noch weitere Antibiotika wie Tiamulin oder Minocyclin enthalten.

In DP19602345.9-41 wird der Zusatz von sterilfiltriertem Hefeextrakt zu Medien beschrieben, die anschließend γ-sterilisiert werden können und die Wachstumseigenschaften von nicht γ-sterilisierten Medien beibehalten.

In der Pharmaindustrie werden zur Verwendung in Reinraumbereichen und Isolatoren γ-sterilisierte Medien gewünscht. Diese Reinraumbereiche müssen auf mikrobielle Kontamination überwacht werden, und das Produkt (also das mikrobiologische Nährmedium), mit welchem diese Überwachung durchgeführt wird, sollte selbst keine Kontamination einbringen, sondern steril sein. Mikrobiologische Medien sind nach Autoklavieren und Abfüllen in z. B. Petrischalen oder anderen Agarträger nicht absolut steril, sondern weisen Kontaminationsraten in der Größenordnung von 0,1 bis ca.1% auf. Erst durch eine anschließende γ-Sterilisierung (oder β-Sterilisierung) mit energiereicher Strahlung werden die Reste vorhandener Kontaminationen abgetötet und ein steriles Produkt erreicht. Allerdings werden durch die γ-Sterilisierung viele

Antibiotika zerstört, so dass anschließend bei Selektivmedien für Hefen und Pilze, deren Selektivität auf dem Zusatz von Antibiotika beruht, diese Selektivität nicht mehr vorhanden ist. So wird z. B. die antibakterielle Wirkung von Chloramphenicol und Chlortetrazyklin durch γ-Bestrahlung mit 25 kgray völlig zerstört und die antibakterielle Wirkung des nur noch schwach wirksamen Penicillins weitgehend reduziert.

Aufgabe der Erfindung war es, ein Verfahren zu finden, das zum selektiven Nachweis von Hefen und Pilzen selektive Antibiotika-Zusätze verwendet, die das Wachstum der Begleitflora auch noch nach γ-Sterilisierung hemmen, ohne einen negativen Einfluss auf das Wachstum von Hefen und Pilzen zu haben.

Diese Aufgabe wird erfindungsgemäß durch ein Verfahren gelöst, das das
a) Zusetzen von Ciprofloxacin zu einem Kulturmedium und anschließendes
b) γ-Bestrahlen des Ciprofloxacin enthaltenden Kulturmediums umfasst. Der Bereich der Ciprofloxacinkonzentration liegt dabei im Bereich von 2 mg/L bis 200mg/L. Das Kulturmedium zur Verwendung im erfindungsgemäßen Verfahren kann zusätzlich andere Antibiotika enthalten, wie eine Kombination aus Ciprofloxacin und Streptomycin oder gegebenenfalls einer Kombination aus Ciprofloxacin und anderen Antibiotika.

### Beispiel 1

Zu Sabouraud Dextrose Agar pH7,0 (Emmons Modification Difco Manual) werden nach Autoklavieren 50 mg/L Ciprofloxacin und 50 mg Streptomycin sterilfiltriert hinzugesetzt. Weiter werden 100 ml einer bei Raumtemperatur sterilfiltrierten Lösung von rohem, nicht autoklaviertem Hefeextrakt zugesetzt. Der Hefeextrakt wird mit einer Suspension von 25 g/Liter (2.5 g/100 ml) Hefeextrakt zur Sterilfiltration angesetzt wie in DP196345.9 beschrieben. Der Agar wird in Petrischalen oder flexible Kunststoffträger (Oberflächenkeimindikator OKI Biotest bzw. Luftkeimindikator LKI Biotest) gegossen und anschließend mit 15-25 kgray γ-bestrahlt.

### Beispiel 2

Zu Sabouraud Dextrose Agar pH 7,0 (Emmons Modification, Difco Manual) werden 50 mg/L Ciprofloxacin zugesetzt und das Medium einschließlich Ciprofloxacin autoklaviert. Nach Autoklavieren wird wie in Beispiel 1 sterilfiltrierter Hefeextrakt zugesetzt. Abfüllung und γ-Bestrahlung erfolgen wie in Beispiel 1.

### Beispiel 3

Zu Sabouraud Dextrose Agar, wie in Beispiel 1, werden 50 mg/L Penicillin V und 50mg/L Streptomycin sterilfiltriert zugesetzt (anstelle Ciprofloxacin und Streptomycin). Abfüllung und γ-Bestrahlung erfolgen wie in Beispiel 1.

### Beispiel4

Zu Sabouraud Dextrose Agar, wie in Beispiel 1, werden 50 mg/L Chloramphenicol und 50 mg/L Chlortetrazyklin sterilfiltriert zugesetzt (anstelle Ciprofloxacin und Streptomycin). Abfüllung und γ-Bestrahlung erfolgen wie in Beispiel 1.

### Beispiel 5

Zu Sabouraud Dextrose Agar, wie in Beispiel 1, werden 15 mg/L Chloramphenicol und 15mg/L Gentamycin sterilfiltriert zugesetzt (anstelle von Ciprofloxacin und Streptomycin). Abfüllung und γ-Bestrahiung erfolgen wie in Beispiel 1.

### Beispiel 6

Rose Bengal Agar von Difco wird nach Difco Manual angesetzt und 50 mg/L Chloramphenicol sterilfiltriert zugesetzt. Die Abfüllung erfolgt wie in Beispiel 1, jedoch folgt keine anschließende γ-Sterilisation.

### Beispiel 7

Rose Bengal Agar von Difco wird nach Difco Manual angesetzt und 50 mg/L Streptomycin sterilfiltriert zugesetzt. Die Abfüllung erfolgt wie in Beispiel 1, jedoch folgt keine anschließende γ-Sterilisation.

### Beispiel 8

Sabouraud (Emmons Modification, Difco) wird nach Difco Manual autoklaviert. Danach werden 50 mg/L Chloramphenicol und 50mg/L Chlortetrazyklin zugesetzt. Das Medium wird in Kunststoffträger (LKI Biotest) abgefüllt, jedoch nicht γ-sterilisiert.

Die nach den vorstehenden Beispielen hergestellten Kulturmedien wurden auf ihre Wirksamkeit gegenüber Bakterien, Hefen und Pilzen untersucht. Die Ergebnisse sind in der Tab. 1 zusammengefasst. Wie sich daraus ergibt, werden grampositive Keime (*B*. *subtilis, S. aureus)* gut gehemmt von Medium 8, von den gramnegativen (*E*. *coli, P. aeruginosa*) wird *P*. *aeruginosa* von Medium 8 nur unvollständig gehemmt, das erfindungsgemäße Medium nach Beispiel 1 hemmt alle bakteriellen Prüfkeime komplett, während das Medium nach Beispiel 4 (entspricht Beispiel 8, jedoch bei Beispiel 4 zusätzlich γ-Bestrahlung) keine Hemmung mehr für alle bakteriellen Prüfkeime zeigt.

Auf Selektivnährböden zum Wachstum von Hefen und Pilzen sollten nur Hefen und Pilze wachsen, jedoch keine Bakterien. Durch die Antibiotika in den unbestrahlten Medien gemäß Beispiel 5-8 werden Bakterien (außer *P. aeruginosa*) gehemmt, Pilze und Hefen zeigen bei Beispiel 8 gutes Wachstum, während bei Beispiel 6 und 7 Rose Bengal (verhindert "spreading" bei Pilzen und wirkt etwas antibiotisch) nicht alle Pilze gut wachsen. Nach γ-Bestrahlung haben nur noch die Medien mit Ciprofloxacin gemäß Beisp. 1 und 2 die erwünschten Eigenschaften (kein Bakterienwachstum auch bei hohen aufgeimpften Keimzahlen, gutes Anwachsen von Pilzen und Hefen auch bei niedrigen Keimzahlen). Nach γ-Bestrahlung (Beispiel 3-5) haben die Medien ohne Ciprofloxacin die erwünschten Eigenschaften verloren und die bakterielle Flora wächst voll durch. Diese erwünschten Eigenschaften sind bei den Ciprofloxacin enthaltenen Medien daruber hinaus über eine Laufzeit von 7 Monaten stabil während sogar die unbestrahlten Medien nach Beispiel 8 bereits nach 3 Monaten in ihrer Hemmwirkung auf Bakterien noch weiter nachlassen.

In weiteren Versuchsreihen wurden die Kulturmedien nach Beispiel 1, 4 und 8 auf ihre Wirksamkeit als selektives Nachweismedium untersucht.

Luftkeimindikatorenstreifen (Agarstreifen LKI von Biotest) werden mit Medium nach Beispiel 1 und nach Beispiel 8 befüllt. Danach werden in einem Raum an 5 aufeinanderfolgenden Tagen unter Verwendung von zwei Luftkeimsammelgeräten des Typs RCS Highflow von Biotest jeweils zehn Messungen pro Tag vorgenommen. Die Messgeräte werden parallel in 1 m Abstand aufgestellt und in einem Gerät mit Agarstreifen LKI mit Medium nach Beispiel 8, das andere mit Agarstreifen LKI mit Medium nach Beispiel 1 versehen. Gleichzeitig werden je 600 L Luft gesammelt. Danach wird die Position der zwei Geräte ausgetauscht für die zweite Messung etc. bis zur zehnten Messung. Die Streifen werden bei 22,5°C ± 2,5°C bebrütet und die gewachsenen Kolonien nach drei Tagen ausgewertet. Kolonien, die kein typisches Pilzaussehen zeigen, werden einer Gramfärbung unterzogen und mikroskopisch ausgewertet, um mögliche Bakterien bzw. Hefenkolonien zu differenzieren. Hefen werden den Pilzen zugerechnet, Bakterien werden extra gezählt und erfasst.

Weiter werden Medien nach Beispiel 1, 4 und 8 in Contact Slides Biotest gefüilt. Auf ungesäuberten Oberflächen werden parallel Abklatsche mit diesen Contact Slides durchgeführt unter Verwendung eines Anpressdrucks von 500 g pro Contact Side (Verwendung eines 500 g Gewichts zum Beschweren der Contact Slides)

Nach Bebrütung für drei Tage bei 22,5°C ± 2,5°C wird analog zu den Luftkeimindikatorstreifen ausgewertet.

Die in Tabelle 2 zusammengefassten Ergebnisse zeigen, dass bei den Luftkeimmessungen sowohl Medium 1 wie Medium 8 das Wachsen von bakterieller Begleitflora verhindern. Dies ist zu erwarten, da hauptsächlich grampositive Keime als Luftkeime vorkommen, gramnegative Keime werden selten aus der Luft isoliert (üblicherweise nur unter Sonderbedingungen wie in Tierställen, Abfallsortieranlagen kommt es zur Isolierung gramnegativer Keime aus der Luft).

Dementsprechend werden mit dem Medium gemäß Beispiel 1 trotz γ-Bestrahlung nach Abklatsch wie gewünscht nur Pilze ohne bakterielle Kontaminanten isoliert, während auf Medium 8 zahlreiche Bakterienkolonien noch durchwachsen und auf der γ-bestrahlten Version von Medium 8 (dies ist Medium Beispiel 4) sogar überwiegend Bakterien wachsen. Damit zeigt das γ-bestrahlte Medium nach Beispiel 1 die gleiche gute Ausbeute an Hefen und Pilzen wie ein nicht γ-bestrahltes Standardmedium auf Sabouraud Basis (Beispiel 8) und ein deutlich besseres Ergebnis als nicht γ-bestrahltes Rose Bengal Medium nach Beispiel 6 und 7.

Alle γ-bestrahlten Sabouraud Medien mit Standardantibiotika nach Beispiel 3-5 hemmen jedoch die bakterielle Begleitflora nicht mehr und sind nicht mehr selektiv nur die erfindungsgemäße Verwendung von Ciprofloxacin, ggf. in Kombination mit weiteren Antibiotika (Beispiel 1 und 2) hemmen auch nach γ-Bestrahlung die Begleitflora bei gleichzeitig unvermindert gutem Wachstum von Hefen und Pilzen.

**Tabelle 1**

| | **Keim** | **Wachstum auf Agar gemäß:** | | | | | | | |
|---|---|---|---|---|---|---|---|---|---|
| | | **Beispiel 1** | **Beispiel 2** | **Beispiel 3** | **Beispiel 4** | **Beispiel 5** | **Beispiel 6** | **Beispiel 7** | **Beispiel 8** |
| Bakterien Inokulum 10⁶ Keime und 10³ Keime | *E. coli ATCC8739* | kein Wachstum | kein Wachstum | gutes Wachstum | gutes Wachstum | Wachstum | gehemmt | gehemmt | gehemmt |
| | *B. subtilis ATCC6633* | kein Wachstum | kein Wachstum | gutes Wachstum | gutes Wachstum | Wachstum | gehemmt | gehemmt | gehemmt |
| | *S. aureus ATCC6538* | kein Wachstum | kein Wachstum | gutes Wachstum | gutes Wachstum | Wachstum | gehemmt | gehemmt | gehemmt |
| | *P. aeruginosa ATCC9027* | kein Wachstum | kein Wachstum | gutes Wachstum | gutes Wachstum | Wachstum | Wachstum* | Wachstum* | Wachstum* |
| Hefen 10.100 Keime | *C. albicans ATCC10231* | gutes Wachstum | gutes Wachstum | gutes Wachstum | gutes Wachstum | gutes Wachstum | gutes Wachstum | gutes Wachstum | gutes Wachstum |
| | *S. cerevisia ATC9763* | gutes Wachstum | gutes Wachstum | gutes Wachstum | gutes Wachstum | gutes Wachstum | wenig Wachstum | gutes Wachstum | gutes Wachstum |
| Pilze Inokulum 10-100 Keime | *A. niger ATCC9642* | gutes Wachstum | gutes Wachstum | gutes Wachstum | gutes Wachstum | gutes Wachstum | Wachstum | Wachstum | gutes Wachstum |
| | *C. herbarum DSM63422* | Wachstum | Wachstum | Wachstum | Wachstum | Wachstum | gehemmt | gehemmt | Wachstum |
| | *F. roseum DSM3019* | gutes Wachstum | gutes Wachstum | gutes Wachstum | gutes Wachstum | gutes Wachstum | gehemmt | gehemmt | gutes Wachstum |
| | *M. racemosus ATCC42647* | gutes Wachstum | gutes Wachstum | gutes Wachstum | gutes Wachstum | gutes Wachstum | gutes Wachstum | gutes Wachstum | gutes Wachstum |
| | *P. chrysogenum ATCC10106* | gutes Wachstum | gutes Wachstum | gutes Wachstum | gutes Wachstum | gutes Wachstum | gutes Wachstum | gutes Wachstum | gutes Wachstum |
| | *S. chartarum ATCC16026* | Wachstum | Wachstum | Wachstum | Wachstum | Wachstum | gehemmt | gehemmt | gehemmt |
| | γ-Bestrahlung | ja 25 KGray | ja 25 KGray | ja 25 KGray | ja 25 KGray | ja 25 KGray | nein | nein | nein |
| | verwendete Antibiotika | Ciprofloxacin + Streptomycin | Ciprofioxacin | Penicillin V + Streptomycin | Chloramphenicol + Chlortetrazyklin | Chloramphenicol + Gentamycin | Chloramphenicol + Rose Bengal | Streptomycin + Rose Bengal | Chloramphenicot + Chlortetrazklin |

| | | | | | | | | | |
|---|---|---|---|---|---|---|---|---|---|
| *Inokulum 10³ gehemmt Wachstum bei 10⁶. Nach 3 Monaten Lagerung auch Wachstum vorn 10³ | | | | | | | | | |

**Tabelle 2**

| **Art der Messung** | **Medium nach Beispiel 8** | **Medium nach Beispiel 1** | **Medium nach Beispiel 4** |
|---|---|---|---|
| 5 x 10 Messungen mit je 600 L Luft in parallel aufgestellten RCS Highflow Luftkeimsammlern von Biotest | insgesamt 4022 Pilze auf 50 Streifen (= 100 % Ausbeute) im Mittel 80 pro Agarstreifen keine bakt. Kontaminationen | insgesamt 4384 Pilze auf 50 Streifen (= 109 % Ausbeute bez. auf Medium nach Beisp. 8) im Mittel 88 pro Agarstreifen keine bakt. Kontaminationen | nicht durchgeführt |
| 3 x 10 Oberflächenabklatsche von ungereinigten Oberflächen parallel mit Medium nach Beispiel 8 und Beispiel 1 in Contact Slides Biotest abgefüllt | insgesamt 1587 Pilze + 117 Bakterienkolonien | insgesamt 1692 Pilze + 0 Bakterienkolonien | insgesamt 1076 Pilze + 1468 Bakterienkolonien |

## Patentansprüche

1. Verfahren zum selektiven Nachweis von Hefen und Pilzen, umfassend:
a) Zusetzen von Ciprofloxacin zu einem Kulturmedium und anschließend
b) γ-Bestrahlen des Ciprofloxacin enthaltenden Kulturmediums.

2. Verfahren nach Anspruch 1, **dadurch gekennzeichnet, dass** die Konzentration des Ciprofloxacin im Kulturmedium 2 mg/L bis 200 mg/L beträgt.

3. Verfahren nach Anspruch 1 und 2, **dadurch gekennzeichnet, dass** es zusätzlich andere Antibiotika enthält.

## Claims

1. A method for the selective detection of yeast and fungi, comprising:
a) adding ciprofloxacin to a culture medium and subsequently
b) γ-radiating said ciprofloxacin containing culture medium.

2. The method according to claim 1,
**characterized in that** the concentration of ciprofloxacin in the culture medium is 2 mg/L to 200 mg/L.

3. The method according to claims 1 and 2,
**characterized in that** it additionally comprises other antibiotics.

## Revendications

1. Procédé pour la mise en évidence sélective de levures et de champignons comprenant :
a) l'addition de ciprofloxacine à un milieu de culture puis
b) l'irradiation y du milieu de culture contenant de la ciprofloxacine.

2. Procédé selon la revendication 1, **caractérisé en ce que** la concentration de la ciprofloxacine dans le milieu de culture est 2 mg/L à 200 mg/L.

3. Procédé selon les revendications 1 et 2, **caractérisé en ce qu'**il contient en outre d'autres antibiotiques.
